(19) **Europäisches Patentamt**
**European Patent Office**
**Office européen des brevets**

(11) **EP 3 740 187 B1**

(12) **FASCICULE DE BREVET EUROPEEN**

(45) Date de publication et mention de la délivrance du brevet:
**10.12.2025 Bulletin 2025/50**

(21) Numéro de dépôt: **18811059.7**

(22) Date de dépôt: **30.10.2018**

(51) Classification Internationale des Brevets (IPC):
**A61K 8/44** *(2006.01)*    **A61Q 5/10** *(2006.01)*
**A61K 8/97** *(2017.01)*    **A61K 8/9789** *(2017.01)*

(52) Classification Coopérative des Brevets (CPC):
**A61K 8/97; A61K 8/44; A61K 8/9789; A61Q 5/10;**
A61K 2800/84; A61K 2800/88

(86) Numéro de dépôt international:
**PCT/FR2018/052694**

(87) Numéro de publication internationale:
**WO 2019/086805 (09.05.2019 Gazette 2019/19)**

(54) **COLORANTS NATURELS INSTANTANES ET LEURS PROCEDES DE PREPARATION**

NATÜRLICHE INSTANTFARBSTOFFE UND VERFAHREN ZUR HERSTELLUNG DAVON

INSTANTANEOUS NATURAL COLORANTS AND PROCESSES OF MAKING THE SAME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.10.2017  FR 1760231**

(43) Date de publication de la demande:
**25.11.2020  Bulletin 2020/48**

(73) Titulaire: **Meneses Da Silva, Edna**
**75013 Paris (FR)**

(72) Inventeur: **Meneses Da Silva, Edna**
**75013 Paris (FR)**

(56) Documents cités:
**EP-A1- 0 729 743    WO-A2-2012/033536**
**CN-A- 105 622 658    CN-A- 107 157 878**
**FR-A1- 2 734 572    JP-A- H0 299 563**
**JP-A- S5 150 938    US-A- 5 908 650**

• **FORESTIER J P ET AL: "HENNE. ABSORPTION DE LA LAWSONE PAR LE CHEVEU//HENNA. SORPTION OF LAWSONE BY HAIR", INTERNATIONAL JOURNAL OF COSMETIC SCIENCE, KLUWER ACADEMIC PUBLISHERS, DORDRECHT, NL, vol. 4, 1 January 1982 (1982-01-01), pages 153 - 174, XP008035325, ISSN: 0142-5463**

• **VANESSA: "FAQ: What is the fastest way to achieve henna colour?", 23 October 2015 (2015-10-23), XP002778231, Retrieved from the Internet <URL:http://naturalhairuganda.com/faq-fastest-way-to-achieve-henna-colour/> [retrieved on 20180215]**

• **CEMIL IBIS ET AL: "Synthesis of novel 1,4-naphthoquinone derivatives: antibacterial and antifungal agents", MEDICINAL CHEMISTRY RESEARCH, vol. 22, no. 6, 2 November 2012 (2012-11-02), pages 2879 - 2888, XP055151872, ISSN: 1054-2523, DOI: 10.1007/s00044-012-0300-y**

• **MOHD YUSUF ET AL: "Assessment of colorimetric, antibacterial and antifungal properties of woollen yarn dyed with the extract of the leaves of henna ()", JOURNAL OF CLEANER PRODUCTION, ELSEVIER, AMSTERDAM, NL, vol. 27, 3 January 2012 (2012-01-03), pages 42 - 50, XP028459159, ISSN: 0959-6526, [retrieved on 20120110], DOI: 10.1016/J.JCLEPRO.2012.01.005**

## Description

L'invention se rapporte au domaine des colorants, en particulier à celui des colorants naturels. Plus particulièrement, l'invention concerne des produits colorants obtenus par réaction d'extraits naturels contenant des précurseurs de colorants (substances colorantes et/ou copigments), leur procédé de préparation, de conditionnement ainsi que leur application. Ces produits colorants peuvent être utilisés dans le domaine cosmétique, en particulier pour teindre des cheveux et tatouer la peau, pharmaceutique/dermocosmétique, agroalimentaire, dans le domaine vétérinaire et pour teindre les textiles. Ces colorants peuvent se présenter sous la forme de poudres instantanées ou de préparations prêtes à l'emploi.

## ETAT DE LA TECHNIQUE

### *Les colorations capillaires*

Il existe deux méthodes principales pour colorer les cheveux humains, les colorations d'oxydation dites « permanentes » et les colorations directes dites « semi-permanente ».

Dans les deux cas, les composants actifs sont des molécules obtenues par synthèse.

Les colorations d'oxydation consistent à mettre en œuvre une ou plusieurs bases d'oxydation localisées dans les fibres capillaires, éventuellement en association avec des coupleurs. Les techniques actuelles proposent l'activation des précurseurs de coloration par l'utilisation de catalyseurs enzymatiques (polyphénoloxydases) ou d'oxydants chimiques (peroxyde d'hydrogène). Cependant, l'utilisation de ces composés pose des risques toxicologiques. Récemment, les risques associés aux colorations "chimiques" pour la santé et l'environnement ont conduit l'Union Européenne à interdire toute une série de colorants.

Les colorations directes comprennent l'application de molécules colorées et colorantes ayant une affinité par les fibres capillaires et les kératines (cheveux, sourcils, poils). Les molécules employées restent plutôt en surface et pénètrent peu à l'intérieur de la fibre.

L'avantage principal de ce type de coloration est de limiter la dégradation des fibres et les risques d'intolérance.

Les extraits de plantes à pouvoir colorant sont utilisés dans cette deuxième catégorie ; elles mettent en œuvre des composés ayant plus ou moins d'affinité pour les fibres capillaires. Les moyens mis en place dans les colorations végétales peuvent être très variés.

### *Les colorations capillaires végétales*

Il existe une demande croissante des consommateurs pour des colorants cosmétiques, textiles ou alimentaires à partir des substances d'origine naturelle, notamment pour éviter les problèmes de toxicité et risques associés aux molécules de synthèse. On constate d'ailleurs que le marché des colorants artificiels est en baisse tandis que celui des colorants naturels augmentent d'environ 10% par an.

L'utilisation des plantes en tant qu'agent colorant se retrouve dans un grand nombre de savoirs traditionnels. Par exemple, le henné (*Lawsonia inermis*) est parmi les plantes colorantes les plus utilisées.

La coloration capillaire végétale d'utilisation traditionnelle fait appel à une composition simple : des extraits des plantes tinctoriales et de pigments (henné, garance, camomille, indigo...) broyés et mélangés à de l'eau. Contrairement à la coloration chimique (d'oxydation) qui ouvre les écailles, éclaircit la mélanine puis, la colore suite à une réaction chimique, les colorants à base de plantes ne pénètrent pas à l'intérieur des fibres. En effet, les colorants contenus dans les plantes adhèrent à la fibre capillaire et ne modifient pas les mélanines naturelles des cheveux. C'est une coloration ton sur ton, qui ne peut pas éclaircir les cheveux. Son effet est semi-permanent (2 à 6 semaines). Malgré leur faible tenue, les avantages des colorants naturels sont multiples : Le résultat couleur est très naturel, sans effet « casque » car les pigments fusionnent avec la couleur de base des cheveux. La repousse est, de ce fait, moins visible. Les allergies sont aussi moins fréquentes qu'avec la technique chimique.

### *Les plantes à pouvoir colorant*

Pour que les colorants d'origine naturelle (végétal, minéral, animal) de l'état de la technique soient efficaces, la matière à colorer (fibres kératine des cheveux, fibres textiles ou autres) est généralement saturée avec un agent « mordant », qui peut être incorporé dans la solution contenant les colorants. Les mordants sont des composés qui adhèrent aux fibres de façon à permettre l'adhésion de l'agent colorant. Les principaux mordants sont à base de chrome, de sels de cuivre, d'étain, de sulfate de fer, d'alun et d'acide oxalique. Cette technique est appliquée depuis l'antiquité pour l'application des teintures sur les fibres végétales (laine, soie, lin).

Différentes plantes peuvent être utilisées pour la coloration des fibres capillaires telle que le henné, l'indigo, le brou de noix, la garance, l'annato, la carmine etc.

Le henné (*Lawsonia inermis L., L. alba*) est utilisé depuis cinq millénaires comme agent colorant pour les cheveux et le corps (Food and Chemical Toxicology 42:517-543, 2004). Un cataplasme des feuilles broyées de henné procure une coloration rousse, orangée ou cuivrée, en fonction de la couleur des cheveux. Le principal constituant colorant du henné est le lawsone (2-hidroxi-1,4-naftoquinona) (CAS 84.988-66-9), présent naturellement jusqu'à 2% de l'extrait brut et sous forme d'hennoside (glucoside).

Dans le cas du henné, l'hydrolyse enzymatique, favorisée par les formulations, conduit au clivage de l'hennoside avec libération de la molécule lawsone, colorant actif mais instable. La poudre de henné doit rester dans une solution aqueuse et légèrement acide entre 6 et 24 heures afin de libérer le lawsone, permettre son utilisation en tant que colorant. D'autre part, le pH de la solution colorante influence le résultat de la coloration. Ainsi, pour un pH en dessous de 6 la couleur prédominante est le rouge. Au-dessus de 6, l'intensité diminue et la couleur jaune devient prédominante.

Les poudres colorantes sont souvent préparées sous forme de cataplasme. Les poudres de henné (de couleur verte) sont en général moulues et présentées avec une granulation très fine qui, mélangé à l'eau, constitue le cataplasme appliqué directement sur les cheveux. Le henné pur ne donne jamais une couleur noire, mais cuivrée-orangée. Néanmoins, certaines caractéristiques, intrinsèques aux extraits bruts ou aux composés colorants, peuvent ne pas être compatibles avec d'autres colorants ou copigments.

L'utilisation croissante du tatouage (Body art) au henné peut également présenter un risque pour la santé, du fait des composés chimiques synthétiques utilisés de façon frauduleuse pour diminuer le temps de brunissement de la peau ou pour potentialiser ou modifier les couleurs (Contact Dermatitis 55:26-29, 2006). En effet, les poudres de hennés frelatés destinées à la coloration des cheveux ou au tatouage temporaire échappent très souvent aux contrôles habituels et certains ingrédients toxiques peuvent être rajoutés. Les hennés foncés (noir ou acajou) peuvent ainsi contenir de la para-phénylène diamine (PPD), un colorant de synthèse, ou dans le pire des cas de l'acétate de plomb, formellement interdit dans l'Union Européenne. Les conséquences peuvent être de graves (par exemple : réactions allergiques au henné pour tatouage frelaté à la PPD). Ainsi, quelques produits vendus sous le nom de « henné » contiennent d'autres colorants conduisant à des résultats inattendus et parfois à une toxicité due à des composés intégrés.

### Exemples de préparations traditionnelles : cataplasmes

Formulation à base de Henné : Les procédés de coloration à partir du henné sont délicats à mettre en œuvre. Une formulation traditionnelle à base de henné est préparée à partir de 30 à 100g de poudre de feuilles broyées (35 à 80 mesh) dissous dans 300 mL d'eau bouillante. Après refroidissement, le cataplasme (ou pâte contenant des matières solubles et insolubles), est appliqué directement sur la chevelure pendant (3 à 24 heures). La chevelure est couverte avec un plastique ou tissu afin d'éviter de tâcher les vêtements. La mauvaise consistance de ce cataplasme conduit généralement à une imprégnation non homogène des cheveux et un résultat non satisfaisant.

Formulation à base d'indigotier : Les cataplasmes préparés à partir de broyats de feuilles d'indigotier (*Isactis tinctoria*) sont d'abord mis à fermenter dans l'eau chaude afin de libérer l'indigo de couleur mauve. Ce cataplasme (dilué ou non) est directement appliqué sur les fibres de kératine. La mauvaise consistance de la composition empêche une imprégnation satisfaisante du colorant conduisant à des colorations non homogènes.

Formulation tatouage (body art) : Pour le tatouage du corps, les feuilles séchées et pulvérisées sont mélangées avec une solution légèrement acide (jus de citron ou orange, vinaigre) afin d'obtenir une pâte qui est appliqué directement sur le corps avec des outils différents. Plusieurs facteurs conduisent à des couleurs qui peuvent varier de orange à auburn.

Il est à noter que l'extrait du henné (Lawsonia Inermis) est non seulement un agent de coloration, mais il procure également de nombreux bienfaits pour la santé. La plante contient notamment des terpénoïdes (effets antimicrobiens), des stérols, des dérivés aliphatiques, des xanthones (colorants jaunes au pouvoir antioxydant et anti-inflammatoire), des tannins, des coumarines, des flavonoïdes, des acides gras, des acides aminés, des huiles essentielles et d'autres constituants. Le Comité Scientifique pour la Sécurité des Consommateurs (SCCS) a conclu sur la sécurité d'utilisation du henné (*Lawsonia inermis, avec* teneur de 1,4% de lawsone) en tant que colorant dans les doses habituelles (100 g de henné/300mL de $H_2O$ ou 330 mg/mL (COLIPA n° C169, 19.09.2013).

Le henné possède ainsi des propriétés antioxydantes, antimicrobiennes, antivirales, antiparasitaires, antifongiques, anti-inflammatoires, antidiabétiques, anticancéreuses, hépatoprotectrices et immunomodulatrices.

### Naphtoquinones naturelles

Les naphtoquinones naturelles (*lawsone, juglone, plumbagine, lapachone*) ont une forte activité fongicide et bactéricide (Nat Prod Res. 2014;28(11): 835-7 ; J Agric Food Chem. 2012 Dec 12; Nat Prod Res. 2012;26(12):1119-24; Phytother Res. 2001 Dec;15(8):676-80.). Ces composés présentent une activité protectrice sur la peau. A titre d'exemple, les propriétés bactéricides, fongicides, cytostatiques du lawsone ont été démontrés (Chelossi, E. & Faimali, M. Science of The Total Environment 356, 1-10, 2006 ;Kapadia, J. G. et al. Anti-Cancer Agents in Medicinal Chemistry- Anti-Cancer Agents) 13, 1500-1507, 2013; Yusuf, M. et al. Journal of Cleaner Production 27, 42-50, 2012.

Les résultats des colorations avec ces cataplasmes sont peu maitrisables, le résultat varie en fonction des concentrations et de la qualité des matières première (henné, indigo etc..) ainsi que de la nature des fibres à colorer.

## INCONVENIENTS DE L'ETAT DE LA TECHNIQUE

Bien que de nombreuses colorations à base de colorants naturels soient décrites, par exemple dans le document Forestiert et al., "Henne. Absorption de la lawsone par le cheveau, Int. J. of Cosmetic Sci., Kluver Academic Publishers, Dordrecht, NL, vol. 4, 1 janvier 1982 (1982-01-01), pages 153-174, et/ou disponibles dans le commerce, celles-ci présentent certains inconvénients.

Tout d'abord, on constate que les colorants d'origine naturelle (plantes, légumes, fruits, agrumes, minérale, algues) sont sous-utilisés dû aux difficultés techniques rencontrées pour l'obtention de colorants efficaces :

- Certains constituants actifs sont instables à la lumière, la chaleur, la température, et/ou aux variations de pH.
- La préparation de cataplasmes à partir des extraits bruts de plantes conduit à des consistances non homogènes dûes, en partie, à l'insolubilité d'une partie des matières extraites. Ceci peut conduire à la diminution de l'efficacité résultant d'une distribution non uniforme sur la chevelure, voire à une coloration non homogène de la chevelure.
- La tenue des couleurs dans les cheveux peut être réduite du fait de l'instabilité du colorant à lumière ou à la chaleur ou de l'absence d'affinité par les fibres de kératine de certains colorants naturels.

Malgré la diversité des produits naturels et le potentiel des couleurs que présentent les végétaux, les colorations végétales disponibles actuellement sur le marché ne permettent que des colorations ton sur ton ou avec des reflets. Cela est dû à plusieurs facteurs intrinsèques aux matières premières ou à la nature des constituants actifs, notamment :

- *Concentration des molécules actives* : L'intensité des couleurs obtenues avec les teintures naturelles est limitée du fait du faible taux de constituants actifs dans l'extrait brut ; à titre d'exemple, le henné contient généralement de 0,5 à 1,5% de lawsone.
- *Solubilité partielle des extraits :* Les matières (feuilles, racines, écorces) sont souvent broyées et ensuite transformées en cataplasmes, préalablement à l'application sur les cheveux. L'insolubilité d'une partie de l'extrait perturbe son application sur les cheveux. En plus d'une distribution non uniforme, il peut y avoir une sensation désagréable au toucher. Le résidu insoluble limite le conditionnement.
- *Préparation non homogènes et temps de repos :* La préparation de l'extrait nécessite 30 min à 1 heure, auquel s'ajoute le temps nécessaire pour l'extraction (hydrolyse) des constituants actifs par les enzymes présentes (6 heures à 24 heures), le temps pour préparer le cataplasme ainsi que le temps de pause après l'application sur la chevelure (30 min à 2 heures). Au total, la coloration traditionnelle aux plantes consomme un temps plus long que pour une teinture 'chimique' classique.
- *Stabilité des composés actifs et durée de conservation :* Les techniques d'extraction associant pH trop acide et température élevée favorisent l'hydrolyse. Ces techniques agressives décomposent les substances colorantes, changent leur capacité colorante et leur degré d'assimilation, sans parler de la destruction partielle ou totale de certaines vitamines, composants d'intérêt pour l'action colorante.

Face à ces difficultés d'utilisation, les consommateurs sont à la recherche de produits naturels, plus respectueux de la santé et de la nature, mais efficaces et dont le résultat esthétique est satisfaisant.

Cette demande croissante des consommateurs pour des produits naturels a conduit à l'apparition des colorants pseudo-naturels. Des appellations trompeuses créent la confusion chez les consommateurs en faisant passer des produits utilisant les principes de la coloration chimique pour des colorations naturelles : "Teinture aux plantes", "Couleur-soin aux plantes tinctoriales", "Aux extraits de litchi révélateur", "Enrichi en aloe vera" ou encore "Avec des ingrédients issus de l'agriculture biologique". En général, ces allégations concernent des colorations chimiques qui sont "enrichies" avec une quantité de composants du règne végétal sans aucun ingrédient ayant la capacité d'agir comme colorant. Il s'agit fondamentalement des colorations aux ingrédients actifs (colorants).

Il existe donc un vide technique face à l'attente des consommateurs qui souhaitent des colorants naturels plus stables et efficaces. De cette situation résultent d'une part, le besoin pour les industriels de disposer d'ingrédients actifs naturels, stables et efficaces en tant que colorants, et d'autre part pour les consommateurs de disposer de produits véritablement naturels, respectueux de leur santé, et en même temps efficaces.

## AVANTAGES DE L'INVENTION

L'invention met en œuvre un procédé de préparation de produits colorants naturels instantanés et hydrosolubles à partir de précurseurs organiques non-colorées formant des associations moléculaires ou complexes avec les pigments ou molécules à pouvoir colorant. Il s'agit d'une réaction mettant en jeu des précurseurs de coloration directe et des copigments présents dans des plantes ou extraits d'espèces différentes avec un catalyseur naturel polyfonctionnel. La présence de molécules polyfonctionnelles telles que les aminoacides semble provoquer des réactions entre ces molécules conduisant à la formation d'un colorant plus stable et des couleurs originales dans la solution résultante.

La présente invention propose un nouveau type de colorants, qui peut être 100% naturel, obtenus par une réaction entre des précurseurs de colorants ou autres colorants et des copigments en présence de catalyseurs polyfonctionnels tels que les aminoacides, ainsi que leur utilisation, en particulier pour les colorations capillaires et les tatouages de la peau, mais aussi dans le domaine de l'agroalimentaire, pharmaceutique, vétérinaire et pour la teinture textile. Ces colorants peuvent se présenter sous différentes formes prêtes à l'emploi ou à dissoudre, telles que des poudres instantanées hydrosolubles. L'invention concerne aussi le procédé de préparation de produits colorants, qui peuvent être uniquement constitués d'ingrédients naturels. La préparation de colorant sous forme d'un lyophilisat hydrosoluble permet une plus grande conservation et facilite la préparation in situ de masques prêts à être appliqués.

Ainsi, l'invention répond aux inconvénients de l'état de la technique pour les raisons suivantes :

a. Le procédé selon l'invention est appliqué à des précurseurs de colorants et des copigments en présence des catalyseurs naturels (exemple : des aminoacides, dérivés soufrés de l'ail), ce qui permet une potentialisation de l'action colorante des plantes et de leur mélange, notamment par l'enrichissement des composés actifs (précurseurs de colorants et leurs dérivés ) ;

b. Les colorants de copigmentation obtenus selon l'invention ont une affinité améliorée pour les fibres de kératine, ce qui permet une coloration plus intense ;

c. La stabilité des colorants extraits des plantes est augmentée (couleur plus stable), notamment lorsque les produits colorants se trouvent sous forme de lyophilisat (alternativement de poudre colorante instantané obtenue via la technique de séchage par atomisation)

d. Les produits colorants naturels proposés sont prêts à l'emploi sous forme de colorants naturels instantanés utilisables dans divers domaines tels que la cosmétique, l'agroalimentaire, le domaine vétérinaire et la coloration textile ou en tant qu'adjuvant pharmaceutique.

## DESCRIPTION DETAILLEE DE L'INVENTION

Les inventeurs proposent de nouveaux produits colorants instantanés et hydrosolubles obtenus par réaction entre un précurseur de colorants naturels d'origines diverses (composés I) et un copigment (composé II) avec un catalyseur naturel polyfonctionnel, notamment un aminoacide (composé III).

Un premier objet de l'invention concerne un procédé de préparation d'un colorant instantané et hydrosoluble consistant à faire réagir au moins un composé I et un composé II avec au moins un composé III, lesdits composés I, II et III étant chimiocompatibles entre eux, dans un milieu faiblement acide dont le pH est compris entre 3,5 et 6,5, à une température comprise entre 30°C et 55°C (exceptionnellement jusqu'à 70°C), pendant une durée comprise entre 10 et 45 minutes, dans lequel :

- le composé I est un précurseur de colorant,
- le composé II est un copigment
  les composés I et II étant choisis parmi les naphtoquinones, les quinones, les antraquinones, les indoles, les bis-indoles, les xanthones, les flavonoïdes, les pyranoanthocyanes, les crommènes, les iridoïdes, les préridines, les isoquinolines, les phanoxaline, les phenazines, les polyènes, les caroténoïdes et les chalcones ;
- le composé III est un catalyseur naturel polyfonctionnel choisi parmi les tannins, les acides aliphatiques, les acides aromatiques et les acides phénoliques, les acides organiques, les carbonates, les alcaloïdes, les aminoacides et les alcaloïdes.

Par « précurseur de colorant » au sens de l'invention, on entend un colorant direct ou un pigment.

De préférence, le procédé de préparation d'un colorant instantané et hydrosoluble selon l'invention consiste à faire réagir les parties hydrosolubles des plantes riches en précurseurs de colorants chromophores et/ou groupements auxochromes en tant que sources des composés I, et/ou II et/ou III.

Dans un mode de réalisation particulier du procédé, la température de la réaction est comprise entre 30°C et 55°C, de préférence entre 40°C et 55°C, de manière encore plus préférée entre 45°C et 50°C, pouvant varier en fonction de la nature et stabilité des composés mélangés. La température peut exceptionnellement aller jusqu'à 70°C.

Dans un autre mode de réalisation particulier du procédé, la durée de la réaction est comprise entre 10 et 30 min, de manière préférée entre 10 et 20 min, de manière tout à fait préférée entre 10 et 15 min. Il est également possible de faire réagir plus long temps en fonction de la nature des composés mélangés.

Dans un autre mode de réalisation particulier du procédé, le pH de la réaction est compris entre 4 et 5.

Le produit colorant obtenu par réaction est refroidi sous agitation à température ambiante (typiquement entre 20°C et 25°C).

Dans un autre mode de réalisation, le procédé comprend en outre une étape préalable à la réaction, consistant à extraire le ou les composés I à partir d'extraits de plantes, tel que cela est exposé ci-après.

Dans un autre mode de réalisation, le composé III est un thioaminoacide, par exemple cystéine, méthionine ou glutathion.

Dans un autre mode de réalisation, le procédé comprend, en outre, une étape de congélation des produits colorants obtenus à l'issue de la réaction.

Dans un autre mode de réalisation, le procédé comprend, en outre, une étape de lyophilisation ou de séchage par atomisation des produits colorants, de sorte à obtenir une poudre instantanée hydrophile. La lyophilisation, peut être réalisée soit directement après la réaction, soit après congélation -broyage. Le séchage par atomisation est réalisé juste à partir de la solution liquide issue de la réaction.

L'objectif est de faire interagir ces composés, dans des conditions douces (pH, température) et concentration élevée. En plus de faciliter le contact des plantes ayant des compositions différentes, les catalyseurs (exemple: aminoacides polyfonctionnels) apportent des sites d'ancrage à ces entités et contribuent à la fixation au support final, les fibres kératines.

La copigmentation est un phénomène naturel qui a lieu en solution et pendant lequel les pigments et autres molécules organiques non-colorées forment des associations moléculaires ou complexes avec les pigments ou molécules à pouvoir colorant. Les copigments ont un effet sur l'augmentation de l'intensité des couleurs et sur la stabilisation des molécules colorantes (Markovic et al., 2000).

L'originalité de ce procédé consiste dans le fait de renforcer la stabilité et l'action colorante des colorants naturels grâce à leur réaction avec des copigments en présence d'un catalyseur polyfonctionnels (naturels ou non) ; les inventeurs ont en effet montré que les colorants naturels obtenus sont plus stables et capables de tenir plus longtemps sur les fibres kératines (cheveux, cils, sourcils, poils) et protéines de la peau notamment. Les produits colorants ainsi obtenus appelés « colorants de copigmentation », correspondent à l'association intermoléculaire, intramoléculaire ou des réactions d'addition pouvant se produire quand on mélange les précurseurs de colorants avec des copigments ou extraits des plantes les contenant sous certaines conditions (température, pH, temps), cela en présence d'un catalyseur polyfonctionnel. Le but est d'obtenir des couleurs plus résistantes aux variations de pH, température, solvants et des nouvelles couleurs issues de combinaisons multiples.

Les précurseurs de colorants et copigments sont sélectionnées afin de trouver une application adéquate en fonction des objectifs souhaités.

Dans le procédé de l'invention, les précurseurs de colorants directs présents dans les extraits des plantes (garance, indigo, henné etc...) sont d'abord libérés de leur forme hétéroside (aglycone + glucides) par les enzymes présentes dans l'extrait brut de la plante. L'extraction des copigments des plantes riches en ces matières ainsi que des copigments auxilliares (chataîgnes, noyer, guarana) est fait en milieu hydroalcoolique.

Il est aussi possible d'utiliser des colorants et/ou copigments déjà extraits, voire purifiés, et disponibles dans le commerce pour réaliser une réaction de copigmentation selon l'invention.

Les précurseurs de colorants sont choisis parmi les extraits des plantes, des fruits, de baies, de grains, d'agrumes, de légumes et leur mélange. Ces matières premières contiennent des principes actifs colorants directs libres ou dérivés (glycosides, sels, etc) dont les structures moléculaires sont variées.

Les composés I de l'invention correspondent aux précurseurs de colorants, les composés II correspondent aux copigments et les composés III correspondent aux catalyseurs polyfonctionnels (aussi appelés « copigments polyfonctionnels » ou « copigments auxiliaires »).

Les colorants directs, pigments et copigments naturels d'utilisation traditionnelle, peuvent être choisis parmi les familles moléculaires (colorants ou pigments) décrites ci-après ou leur dérivés (quinones, antraquinones, naphtoquinones, indoles, anthocyanes, xantones, flavonoïdes, cromènes, pteridines, isoquinolines, phenothiazine, iridoïdes, polyènes, chalcones, ainsi que les shikimates, terpenes, steroides, les polyacétates, aminoacides etc...).

Les colorants directs, pigments et copigments sont issus de l'extraction aqueuse (ou hydroalcoolique) des plantes, feuilles ou d'autres parties végétales. Plusieurs plantes ou autres matières d'origines naturelle ayant un pouvoir colorant direct peuvent être utilisées ou modifiés afin d'obtenir les composés I ou II. Les matières sont broyées, macérées, atomisées préalablement à l'extraction aqueuse ou hydroalcoolique. La quantité du précurseur de colorant ou molécules colorantes directes dans l'extrait sec initial doit être suffisante pour obtenir une coloration visible. Cette quantité doit être adaptée en fonction du précurseur et de l'intensité voulue pour la coloration.

D'un point de vue structurel, une molécule de colorant direct organique présente une certaine configuration nécessaire à l'obtention de la couleur. Plusieurs conditions doivent être réunies pour que la molécule fasse apparaître la coloration :

- elle doit porter des groupements chromophores et être fortement conjuguée (alternance de nombreuses doubles et simples liaisons) ;
- elle doit porter des groupements auxochromes qui intensifient la coloration initiale produite.

Ainsi, un colorant direct comporte obligatoirement deux types de groupements : les groupements chromophores et les groupement auxochromes.

Les copigments ne sont pas colorants par eux-mêmes. il s'agit de molécules colorées ou incolores, présentes dans le règne végétal, minéral ou animal, distinctes des molécules à pouvoir colorant. Comparé à un colorant direct, un copigment comprend soit des groupements chromophores, soit des groupements auxochromes.

Une grande variété de plantes présente des constituants (familles de molécules) ayant des structures chimiques et propriétés similaires, notamment des copigments tels que les flavonoïdes (flavones, flavonols, flavanones, and flavanols) et autres polyphénols, les alcaloïdes, les aminoacides, notamment les thio-aminoacides, les acides organiques (i.e. hydroxycinnamic acids and hydroxybenzoic acids) tannins, etc...

Les aminoacides, par exemple, sont considérés comme des molécules à faible toxicité et ils contiennent au moins des groupements fonctionnels carboxyl et amine, et certains présentent des groupes soufre, guanine thiol ou hydroxyles phénoliques.

L'interaction entre les précurseurs de colorants et les copigments confère une plus grande intensité de la solution colorante finale. Ils peuvent par exemple être ajoutés à l'extrait aqueux ou hydroalcoolique du composé colorant ou préparé de façon séparée pour être mélangés à ce composé colorant dans une deuxième phase. Toutefois, le simple mélange des précurseurs de colorants et des copigments ne permet pas d'obtenir des colorations homogènes des supports (exemple : fibres capillaires, tissus). l'invention résout cet inconvénient en proposant d'ajouter un catalyseur naturel à la réaction entre précurseurs de colorants et copigments.

Les composés I et II sont choisis parmi les précurseurs de colorants et copigments naturels tels que décrits ci-après. Ces pigments peuvent être obtenus de sources naturelles, végétales, animales ou minérales. Toutefois, des colorants de synthèse peuvent également être considérés dans cette catégorie.

Les colorants organiques naturels qui suivent sont classés selon leur structure chimique. À l'échelle moléculaire, une certaine configuration structurale de la molécule est nécessaire à l'obtention de la couleur. Les groupes d'atomes responsables de la couleur des colorants sont appelés groupes chromophores. Ces groupes chromophores peuvent renfermer des groupements azoïques s (-N=N-), carbonyles (-C=0), nitrés (-N02+), nitrosés (-N=0), alcènes (-C=C-), etc. Ces doubles liaisons doivent être nombreuses pour que le corps soit coloré.

Les groupes appelés « groupes auxochromes » permettent la fixation des colorants sur les fibres et peuvent modifier la couleur du colorant. Les groupes auxochromes peuvent être acides (-COOH, SO$_3$H, -OH) ou basiques (NH$_2$, NHR, NR$_2$).

Les familles moléculaires et plantes contenant des colorants directs, pigments et copigments utilisés en tant que substances actives dans le cadre de la présente invention sont présentés ci-après :

**Composés I et II : Colorants et pigments directs, et copigments**

- NAPHTOQUINONES ET DERIVES : Noyer ou brou de noix (*Juglans cinerea, J. regia, J. nigra*) : juglone, juglandine ; Henné (*Lawsonia inermis, Lawsonia alba*)*:* lawsone; Orcanette des teinturiers (*Alkanna tinctorial , Lithosfermum tinctorium* (VAH L.) : alkannine; Tabebuia et Tecoma (*Tabebuia sp, T. Avellaneda):* lapachol, beta-lapachone; Droséra (*Drosera rotundifolia, D. intermedia , D. anglica):* plumbagone; Shepherdie du Canada *(Shepherdia canadensis) :* chimaphiline, balsamine (*Impatiens balsamina*).

- QUINONE, ANTRAQUINONE ET DERIVES : Garance (*Rubia tinctorum, R. peregrina, R. cordifolia),* les racines ont jusqu'à 19 colorants : l'alizarine, purpurine, xanthopurpurine, rubiadine, l'acide ruberythrinique ;Rhubarbe, racines (*Rheum rhaponticum, R. palmatum, R. officinale):* l'acide crysophanique ; Gaillet, ou Caille-lait blanc, racines *(Galium mollugo, G. odoratum, G. verum)* : alizarine ; Aloes *(Aloe vera) :* aloïne A et B, aloésone ; Lacca (Coccus laccae): l'acide laccaique A, B, C et D Rouge Carmin (Cochenille de *Coccus cacti* ou *Dactylopius coccus);* utilisé avec ou sans mordant) : acide carminique, l'acide kermesique, l'acide flavokermesique ; Millepertuis (*Hypericum perforatum*) : hyperofrine, hypericine et quinones polycicliques.

- INDOLES, BIS-INDOLOÏDE ET DERIVES : Indigotier, feuilles *(Indigofera tinctoria, Indigo suffraticosa, I. articulata, I. arrecta, I. gerardiana, I. argenta, I. indica, I. longiracemosa, Persicaria tinctoria):* indigo *;* Pastel des teinturiers ou Jaune d'or (Isactis tinctoria) : *cis- et trans-indigo, indigorubine;* Polygonum (*Persicaria tinctoria*) ; Gardena blue (*Clerodendrum trichotomium):* phycocyanolibin, guaiazulène, trichotomine; Beterrave (*Beta vulgaris) :* betanines, betanidine, isobetanidine, vulgaxanthines (le rouge de betterave est très instable) ;Melanines des plantes : allomelanines et phaeomelanines.

- XANTONES, FLAVONOIDES, ANTHOCYANES ET DERIVES : Extraits de rose, thé et fruits : pomme, raisin, mûres, açai: pélargonidine (Pelargonium), cyanidine (roses, cramberries), malvidine, paeonidine, delphinidine (*Viola tricolor*), catequine (*Acacia catechu*) ; Chamomille, fleurs (*Matricaria recutila, M. chamomilla*) : azulene, apigenine ; Bourdaine, écorces *(Rhamnus frangula, Frangula alnus) : flavonoides, tanins;* Açaí, fruits (*Euterpe oleracea*) : Antocianines, polifenois et tanins ; Œillet *(Dianthus caryophyllus) :* cyanidine, pélargonidine; *Raisin rouge (Vitis vinifera):* cianidine et autres polyphénols (tanins, pepins polyphenoliques) ; *Marc de raisin :* lignane (lyonirésinol) et autres polymères ; Sophora, boutons floraux (*Sophora japonica) :* rutoside ; Ecorces de chêne, thé, noix, oignons rouges : quecetine, hesperidine, epicathequine, gallocatequine; Les baies colorées et noires (*Ampelopsis glandulosa, Ribes nigrum, Vaccinium vitis-idaeae, Rubus ideaeus, Fragaria ananassa, Rubia tinctoria) ;* Pyranoanthocyanines naturelles: adducts malvidin 3-glucoside pyruvique, peonidin 3-glucoside, 4-vinylcatechin, vitisine-B; Anthocyanines polyacylés (*Cliteria ternatea, Vitis vinifera, Rosa hybrida*) *:* ternatines A1-3, B1-4, C1-4 et D1-3, tecophiline, cyanodelphinidine, phacelianine, alatanine C, rosacyanine B.

- CROMENES, IRIDOÏDES ET DERIVES : Bois de campêche, écorces et bois (*Haematoxylon campechianum*) : haematoxyline; Bois du Brésil (*Caesalphinia echinata*) : brasiline, et son dérivé d'oxydation braziléine (solubles dans l'eau) ; Kamala, fruits (*Mallotus philippensis) :* rottlerine ; Santal (*Santalum album*) : santalines, santal (isoflavone) ; Manguier (*M. indica*): mangiferine ; Gentiana (*Gentiana lutea*) : gentisine, oleuropéoside, amarogentioside, sweroside ; Jenipapo ou Jagua, fruits (*Genipa americana*) : genipine.

- PTERIDINE ET DERIVES : Légumes, brocolis, épinard, noix, avocat, grains complets : riboflavine bioactive (jaune fluorescent).

ISOQUINOLINE ET DERIVES : Sanguinaire du Canada, racines (*Sanguinaria canadensis*) : sanguinarine (1%), berberine.
PHENOXAZINE, PHENAZINE ET DERIVES : Liquen (*Rocellia tinctoria, Ochrolechia tartarea, E. prunesti*) : orceine, orchile

- POLYENES, CAROTENOIDES, TERPENOIDES ET DERIVES : Paprika (*Capsicum annum, Capsicum annuum L.*)*:* capsanthine, Capsorubine; Rocou ou Annato (*Bixa Orellana*)*:* bixine, nor-bixine ; Carrote (*Daucus carota*)*:* alpha, beta et gamma-carotene ; Safran (*Crocus sativus*) : orange crocetine ; Rosa canina: rubixanthine ; Jasmine (*Gardenia jasminoides*) *:* crocine ; Tarragone (Tagetes lucida): lutéine; Orange (*Citrus sinensis*) *:* β-citraurine et violaxanthine ; Curry (*Curcuma longa*) : curcumine ; Coton (Gossypium sp.) : gossypol.

- CHALCONE ET DERIVES : Carthame, fleurs (*Carthamus tinctorium*)*:* safflomine A and B (solubles dans l'eau), carthamine (insoluble dans l'eau)

**Composés III : copigments auxiliaires/polyfonctionnels**

- Tanins : guarana (*Paulinia cupana*)*,* grenade (*Punica granatum*)*,* noix de galles (*Quercus infectoria*)*,* sumac (*Rhus Coriaria*)*,* châtaignes, (*Castanea sativa* )*,* clous de girofle *(Syzygium aromaticum*))*,* feuilles de symplocos, (*Symplocos cochinchinensis), aubépine ; Crataegus mongyna), cyprès et divers* autres tannins condensés ou hydrolysables)].
- Acides aliphatiques : acide citrique (agrumes), acide tartrique (rhubarbe, raisin), acide acétique (vinaigres de raisin, ris), acide malonique, acide succinique, acide ascorbique.
- Acides aromatiques : l'acide caféique (café), acide chlorogénique (chataîgne, thé), acide quinique, acide coumarique, acide synaptique, acide ferullique, acide gallique.
- Acides phenoliques : acide hydroxycinnamique, acide hydroxybenzoïque.
- Carbonates (bicarbonate de sodium, hydrogenocarbonate de sodium ou potassium), l'eau de mer (mélange de sels) ou l'infusion de cendres de bois (qui remplace les carbonates) ; L'alun de potassium ou disulfate d'aluminium et de potassium, les oxydes de fer, de manganèse, ou zinc ;
- Sucres réducteurs : fructose, glucose, rhamnose, galactose, arabinose, xylose and sucrose;
- Composés soufrés (dérivés soufrés de l'ail, thio-aminoacides tels que la cystéine, N-acetyl-cystéine, methionine, glutathion), Composés azotés (hydrolysats de protéines végétales, autolysats de levures, acides aminés).

Les acides aminés sont considérés comme des molécules de faible toxicité et contiennent des groupements fonctionnels comme les soufre, thiol et groupements hydroxyles phénoliques. Tous ces groupements peuvent être liés de façon covalente à d'autres biomolécules comme les protéines (ex. kératines).

Une seule source de colorant, seule ou mélangée à des copigments, peut-être la base d'une grande variabilité chromatique par l'utilisation des copigments auxiliaires, acides, basiques, sels métalliques ou d'autres matières d'origine naturelle.

Les copigments auxiliaires utilisés dans le procédé de copigmentation selon l'invention, sont préférentiellement d'origine végétale. Les plus connus sont le rhyzome de rhubarbe (acide tartrique), les thio-aminoacides (méthionine, glutathion, cystéine ou ses dérivés), l'infusion de cendres de bois (qui remplace les carbonates), les tanins végétaux présents dans les noix de galles, le thé vert, l'écorce de grenade, ou du bois de châtaignier ou encore le Symplocos.

L'acylation par les acides organiques (aromatiques et/ou aliphatiques) est connue pour apporter une meilleure stabilité aux colorants et pigments. L'acide tartrique est utilisé dans l'alimentation comme additif alimentaire (numéro E334[13]), et peut être utilisé comme un régulateur de pH.

Lors de la préparation des colorants selon l'invention, les copigments auxiliaires sont choisis selon les ingrédients actifs présents dans l'extrait de plante puis sont mélangés selon la compatibilité des structures moléculaires de sorte à obtenir selon les cas :

- l'amélioration de la stabilité des composés (pH, température, lumière, chimique),
- l'amélioration de leur solubilité en milieu physiologiquement acceptable,
- l'augmention de l'intensité et/ou la tenue des couleurs finales
- l'augmentation de l'affinité des colorants vis-à-vis des fibres kératiniques (chevelure, barbes, sourcils, des ongles, des pieds et des mains) et/ou de la peau, ou des fibres textiles à colorer.

La combinaison des copigments auxiliaires avec une ou plusieurs molécules choisies parmi les précurseurs de colorants et/ou les copigments peut être choisie par l'homme du métier qui saura établir la biocompatibilité des composés entre eux et choisir les sources de composés et les conditions de la réaction de copigmentation en fonction de la couleur désirée.

Dans un mode de réalisation préféré de l'invention, les composés I, II et III sont des produits d'origine naturelle de sorte à préparer un produit colorant 100% naturel. Toutefois, certains composés d'origine naturelle peuvent être remplacés par des produits de synthèse et être utilisés dans une réaction selon l'invention. De plus, les adjuvants classiques d'origine synthétique utilisés dans la composition des teintures pour les cheveux, la peau ou les textiles peuvent être intégrés à la composition finale des produits selon l'invention sous l'œil attentif de l'homme de métier qui saura les choisir par rapport à leur compatibilité chimique avec des polymères anioniques ou non anioniques, amphotères, des agents épaississants minéraux ou organiques, des antioxydants, des parfums, tampons, dispersants, conservateurs, opacifiants ou des agents de conditionnement divers.

Un deuxième objet de l'invention concerne un colorant de copigmentation obtenu par un procédé de préparation tel que défini précédemment.

Le procédé de préparation de ce colorant met en œuvre des composés I, II et III dans des conditions de réaction douce.

Afin de distinguer la composition de l'invention des produits de l'art antérieur, le produit colorant obtenu par le procédé de copigmentation selon l'invention, est nommé « *Colorant de copigmentation* ».

Dans un mode de réalisation particulier, le colorant de copigmentation se trouve sous la forme d'une solution liquide. Cette solution peut résulter directement des extraits bruts préalablement traités pour obtenir uniquement les substances hydrosolubles.

Dans un autre mode de réalisation particulier, le produit colorant se trouve sous forme congelée. En effet, la solution réactionnelle résultante peut être directement congelée pour conservation.

Dans un autre mode de réalisation particulier, le produit colorant se trouve sous forme d'une poudre instantanée. Cette poudre peut être obtenue par lyophilisation ou par séchage par atomisation, selon les méthodes connues de l'homme du métier. De manière préférée, le produit colorant est un lyophilisat (poudre hydrosoluble).

La lyophilisation est une des meilleures méthodes de séchage puisqu'elle conserve ces caractéristiques organoleptiques, notamment l'odeur et la couleur, et ce pendant de longues périodes, pouvant aller jusqu'à plusieurs années. La réfrigération n'est pas nécessaire pour conserver les produits lyophilisés. Le poids des aliments est aussi diminué par la lyophilisation, ce qui est un avantage pour le transport et le stockage des produits.

Le lyophilisat est préparé directement à partir de la fraction hydrosoluble ou à partir de la fraction hydrosoluble congelée et broyée en petits morceaux.

Les lyophilisats sont des poudres colorantes instantanées présentant des formes (dentelles, étoilés, plaques) qui varient selon la composition des matières premières, et une excellente solubilisation en milieu aqueux. La réhydratation des colorants instantanés est complète par solubilisation en milieu aqueux ou hydroalcoolique, donc beaucoup plus facile à utiliser. Les lyophilisats peuvent être mélangés à des produits de soins (beurre, gel, mousse, crème) afin de préparer les masques colorants pour les fibres kératines. Les couleurs des solutions de départ sont parfaitement maintenues après dissolution. La réhydratation des colorants-pigments lyophilisés est beaucoup plus facile que pour des composés correspondants sous forme déshydratée. Leur structure poreuse rend la réhydratation possible avec de l'eau froide ou chaude, et ce, en quelques secondes. L'aspect visuel et la consistance sont appréciés des consommateurs.

Le catalyseur polyfonctionnel, notamment les aminoacides et en particulier les thiomaninoacides, incorporés dans la le milieu réactionnel apportent une plus grande affinité de la composition hydrosoluble aux fibres capillaires et, *in fine,* une tenue au lavage supérieure aux préparations traditionnelles sous forme de cataplasme. Contrairement aux cataplasmes, les colorants instantanés n'ont pas de parties insolubles dans leurs compositions, ce qui rend leur texture particulièrement agréable et plus facile à conditionner et appliquer.

Dans un mode de réalisation de l'invention particulièrement préféré, le produit colorant est obtenu à partir d'ingrédients 100% naturels. En particulier, un tel produit peut être obtenu à partir d'ingrédient 100% végétaux.

Bibliographie

- M. J Middelveen, P. J Mayne, D. G Kahn, R. B Stricker, Characterization and evolution of dermal filaments, Clin Cosmet Investig Dermatol ; 2013; 6:1-21.

- Middelveen MJ, Rotaru GM, McMurray JL, Filush KR, Sapi E, et al. Canine Filamentous Dermatitis Associated with Borrelia Infection. J Vet Sci Med Diagn 2016 ; 5:6.

- Pradeepa V, Senthil-Nathan S, Sathish-Narayanan S3, et al., Potential mode of action of a novel plumbagin as a mosquito repellent against the malarial vector Anopheles stephensi ; Pestic Biochem Physiol. 2016 Nov;134:84-93.

- Mohd et al. (2012), Assessment of colorimetric, antibacterial and antifungal properties of woollen yarn dyed with the extract of the leaves of henna (Lawsonia inermis), Journal of Cleaner Production 27 (2012) 42.-50

- Scientific Committee on Consumer Safety (SCCS), SCCS/1511/13, OPINION ON Lawsonia inermis (Henna), 19.09.2013

Des exemples de produits colorants selon l'invention sont décrits dans le Tableau 1.

**TABLEAU 1 : Compositions de produits colorants selon l'invention.**

| COLORANTS DE COPIGMENTATION SOUS FORME LIQUIDE | | | |
|---|---|---|---|
| **Composés A\*** (quantité d'extrait brut) | **Composés B \*** | **Couleur obtenue** | **Classification Pantone** |
| Henné (25 à 100 mg/ml) + café (2,5 à 15 mg/ml) | Tanins (25 à 100 mg/ml) + polyphénols (2,5 à 15 mg/ml) + aminoacide (Cystéine : 2,5 à 150 mg) | chocolat | 1405C |
| Henné (25 à 100 mg/ml) + café vert (5 à 250 mg/ml) | Tanins (25 à 100 mg/ml)+ acide polyphénolique (5 à 250 mg/ml) + aminoacide (cystéine : 2,5 à 150 mg/ml) | taupe | 17-0929 |
| Annato (7 à 50 mg/ml) + café vert (5 à 250 mg/ml) | Acide polyphénolique (5 à 250 mg/ml) + aminoacide (csytéine : 2,5 à 150 mg/ml) | mandarine | 13-0720 |
| Henné (25 à 100 mg/ml) +carmin (E120) (5 mg/ml - 300 mg/ml) | Sucres + tanins + aminoacides (cystéine) | Rouge vénitien | 19-1664 |
| henné (25 à 100 mg/ml) + châtaigne (20 mg/ml - 360 mg/ml) | Tanins + aminoacides (cystéine : 2,5 à 150 mg/ml) | Jaune d'or | 14-0848 |
| Henné + vigne | Antocianines (10 - 200 mg/ml) + tanins + aminoacides (cystéine) | lilas | 15-3817 |

COLORANTS DE COPIGMENTATION SOUS FORME DE LYOPHILSAT

| Henné | Tanins | orange | 17-1463/14-0848 |
|---|---|---|---|
| Henné + café vert | Tanins + acide polyphénolique + aminoacide (cystéine) | taupe | 17-0929 |
| Henné + café | Tanins + polyphénols + aminoacide (cystéine) | chocolat | 1405-C |
| Henné + brou noix | Tanins + aminoacide (cystéine) | taupe | 17-0929 |
| Annato + café vert | acide polyphénolique + aminoacide(cystéine) | Jaune primevère | 13-0739 |
| *Soumis à des techniques physiques douces pour l' extraction des ingrédients actifs | | | |

A la lecture du Tableau 1, on constate que :

- Les couleurs obtenues par l'utilisation des précurseurs de colorants individuels sont assez différentes par comparaison aux couleurs obtenues par les colorants de copigmentation;
- Les couleurs obtenues par l'utilisation des colorants de copigmentation sont homogènes, ce qui indique que le résultat de coloration n'est pas associé à la coloration des composants individuels ;

Les poudres instantanées présentent les mêmes couleurs après dissolution que les colorants sous forme liquides. Ceci indique qu'il n'y a pas de perte ou changement des agents colorants lors du processus de filtration, déshydratation et conditionnement ; L'élimination des résidus insolubles des extraits naturels conduit à l'enrichissement des constituants hydrosolubles et par conséquent l'amélioration de la surface de contact entre les molécules actives et les kératines, apportant une meilleure efficacité de coloration.

Les arômes naturels présent dans les extraits des plantes sont maintenus et apportent des caractéristiques organoleptiques notables en fonction des composants I, et II (exemple : curcuma, café, roses).

La présence des copigments (tanins, chalcones, caroténoïdes, acides aliphatiques etc...) stabilise les précurseurs de colorants et en même temps renforce action colorante par un effet de potentialisation des couleurs résultantes et une plus grande variabilité de couleurs.

Sans le catalyseur polyfonctionnel, seul le colorant direct (exemple Lawsone ) imprègne les fibres de façon durable. Les types de constituants des copigments (flavonoïdes, antocyanes, caroténoïdes etc) jouent un rôle important sur la couleur finale des colorants de copigmentation, même si ces copigments ne sont pas directement des agents colorants. Toutefois, l'aminoacide doit jouer un rôle dans l'association entre les molécules colorants/copigments et augmenter l'affinité du colorant final aux fibres capillaires.

La préparation des colorants naturels instantanés (colorants de copigmentation) à partir de lyophilisats est plus facile, plus rapide et l'application plus confortable, avec la possibilité d'utiliser des produits de soin comme les masques de soin par l'addition des alginates (polysaccharides), les beurres (karité) ou encore les conditionneurs comme support d'application.

La non-utilisation des agents agressifs (ammoniaque, peroxyde d'hydrogène, parabènes) permet une conservation de fibres kératines et préserve la santé du cuir chevelu.

La durée de conservation dépend tout particulièrement du mode de conditionnement, mais la plupart des produits alimentaires lyophilisés (légumes, lait, café soluble) convenablement conditionnés ont une durée de conservation très longue.

**Stabilité des composés actifs et durée de conservation.**

La lyophilisation conserve pratiquement intactes toutes les qualités du produit frais (capacités tinctoriales, arômes, fraîcheur). De plus, la lyophilisation conserve les vitamines A, B et C à des taux très proches du produit frais, et cela même après de nombreux mois de stockage. Bien que ce procédé permette la conservation des souches microbiennes, il y a pourtant destruction d'une quantité importante de la population bactérienne de contamination. En conséquence, on obtient une épuration des denrées traitées, se traduisant par une amélioration de la qualité hygiénique.

L'invention concerne également un colorant de copigmentation caractérisé en ce qu'il est instantané, hydrosoluble et qu'il ne comprend pas de fraction insoluble.

Ces colorants de pigmentation sont hydrosolubles, leur composition plus stable que les autres colorants naturels, ils présentent une affinité améliorée pour les fibres de kératine, une tenue plus longue. Les résultats en terme de couleur, sont homogènes, les couleurs sont originales par rapport aux produits naturels de l'état de l'art. De plus un grand nombre de formulations différentes sont possibles grâce au conditionnement au format lyophilisé/desséché.

Un troisième objet de l'invention concerne un kit de coloration capillaire, ce kit comprenant :

- un colorant de copigmentation (lyophilisat) tel que défini précédemment,
- un gel ou un beurre naturel à usage cosmétique
- des ustensiles pour mélanger les composants et appliquer le masque obtenu sur les cheveux.

Le gel ou le beurre se trouvant dans le kit servent de support d'application du produit colorant. Il peut s'agir par exemple, d'un beurre de karité ou d'un gel d'alginates.

Dans un mode de réalisation préféré, le produit colorant est un produit 100% naturel. De même, le gel ou le beurre utilisés comme support au produit colorant sont de préférence des produits d'origine naturelle, pouvant être composés aléatoirement de façon à obtenir des tonalités ou reflets personnalisés.

Ce kit peut, en outre, contenir une notice explicative contenant des instructions relatives à l'utilisation du produit colorant dermo- phyto-pharmaceutique.

Un quatrième objet de l'invention concerne l'utilisation des colorants tels que définis précédemment dans le domaine cosmétique, agroalimentaire, vétérinaire et de la teinture textile.

Les colorants naturels trouvent une application particulière en cosmétique, en réponse à l'attente des consommateurs pour des compositions colorantes naturelles exemptes de produits chimiques, notamment pour colorer les cheveux et tatouer la peau.

L'utilisation les produits colorants instantanés est très simple ; ceux-ci peuvent être mélangés à des produits des soins capillaires. Les alginates naturels (algues), les beurres (karité, murumuru, cacau, etc), les mousses et conditionneurs divers peuvent être utilisés comme support. Néanmoins, les matières hydrophiles sont plus appropriées pour être utilisées comme support des lyophilisats hydrosolubles ; par exemple, les alginates de sodium, polysaccharides obtenus à partir d'algues marines, apportent une belle consistance de gel et intègrent parfaitement les lyophilisats.

L'application des colorants naturels instantanés est plus facile que celle des colorants naturels classiques. Les colorants instantanés peuvent être simplement incorporés à un après-shampooing. Il suffit alors de mélanger le colorant instantané avec l'après-shampoing. Les colorants instantanés peuvent aussi être incorporés dans un masque capillaire, qui sera appliqué sur les cheveux et laissé reposer pendant au moins 30 min. Avant rinçage, les cheveux peuvent être séchés au sèche-cheveux afin d'accélérer la coloration.

En plus du domaine cosmétique, ces colorants instantanés, de préférence 100% naturels, peuvent être utilisés dans le domaine agroalimentaire pour colorer les boissons, bonbons, pâtes etc... ) ou utilisés dans la formulation pharmaceutique.

Dans le domaine de la teinture textile (laines, soie, lin), les colorants instantanés ou poudres séchées peuvent être utilisés pour une teinture textile éco-responsable (ou bio-teintures). Ces colorants naturels peuvent éventuellement être combinés à des teintures non naturelles (teintures semi-synthétiques).

Il est aussi possible d'utiliser les produits colorants selon l'invention dans le domaine vétérinaire pour teindre les poils des chiens, chats et autres animaux domestiques.

En résumé, l'invention apporte les innovations suivantes :

- La stabilisation des matières naturelles colorantes par un procédé faisant réagir des entités chimio-compatibles en présence d'un catalyseur approprié.
- La mise en valeur des colorants et copigments naturels aujourd'hui très peu exploités faute de savoir les stabiliser.
- Un large panel de couleurs chatoyantes obtenues uniquement à partir d'ingrédients naturels, dont l'intensité de couleur est augmentée, voire modulée. On peut parler de « nouvelles couleurs naturelles ». Ces variations des couleurs sont obtenues par mélange de matières colorantes identifiées avec d'autres molécules colorantes d'origine naturelle.
- Ces variations des couleurs sont obtenues par mélange de matières compatibles avec d'autres molécules colorantes d'origine naturelle.
- Un conditionnement innovant pour des colorants prêts à l'emploi par simple solubilisation aqueuse des lyophilisats.
- L'efficacité de la coloration capillaire est optimisée par l'augmentation de la surface de contact entre les agents actifs et les cheveux. En effet, l'utilisation de gels, beurres, crèmes, plutôt qu'un cataplasme apporte un avantage technique et de confort nouveau.

*Usage en dermocosmétique/pharmaceutique*

Les pigments naphtoquinones ont des propriétés multiples (bactéricide, fongicide, répulsive d'insectes, dépoluantes etc), en plus de sa capacité colorante. Ces substances ainsi que ses dérivés actifs, peuvent être utilisés pour le traitement des dermatoses bactériennes chez l'homme (Maladie de Lyme, des dermatites filamenteuses à borréliose), ou dermatoses animales (par exemple, la dermatite digitale bovine ou canine).

Souvent multi-symptomatiques, ces dermatoses sont caractérisées par la formation des filaments accompagnés des éruptions prurigineuses. Des études ont montré que ces filaments peuvent être composés de kératines colorées et collagène.

Un nombre croissant des personnes dans le monde sont affectés par une dermopathie (borreliosis) transmise par les tiques depuis une quinzaine d'années. Ceci est caractérisé par la présence des fibres et filaments issus de la peau et souvent associé à d'autres symptômes (arthralgies, fatigue extrême et fonctions cognitives altérées) (J Dtsch Dermatol Ges. 2010 Apr;8(4):234-42.). Des analyses histologiques et en microscopie électronique ont révélé l'origine de ces filaments. Ils semblent être composés de kératine et collagène, résultat d'une prolifération et activation des kératinocytes et fibroblastes dans l'épiderme. Des spiroquèttes ont été détectées chez ces personnes atteintes (Clin Cosmet Investig Dermatol. 2013;6:1-21).

Les sources de naphtoquinones actives sont variables. La substance juglone est présente dans les extraits de J. regia ou J. nigra (Fischer TC et al., 2012) ; la lawsone présent dans les extraits de Lawsonia inermis (Sritrairat N et al., 2011), la plumbagine identifiée dans Plumbago zeylanica (Pradeepa V. et al., 2016) ainsi que d'autres quinones actives (Futuro et al., 2018). Les naphtoquinones présentent un potentiel comme adjuvants au traitement de ces dermopathies filamenteuses par leur actions bactéricide, fongicide ou répulsive associé à leur affinité naturelle par les fibres de kératines.

*Usage vétérinaire*

De même dans le domaine vétérinaire, la dermopathie digitée bovine est associée à une infection par des spiroquettes. Le stage prolifératif a démontré la formation de filaments de kératine sur la peau et entre les sabots des animaux affectés. L'étiologie de cette dermatite n'est pas encore connue, mais la présence des spirochètes et autres coïnfections ont été impliquées sur la pathogénèse de cette affection vétérinaire (Middelveen MJ1, Stricker RB, 2011).

Une dermatite similaire a été identifié chez les canins, associé à l'infection par Borrelia (Marianne J Middelveen et al., 2016). Des études récentes ont démontré que les bio-fibres de kératine sont produites par les cellules épithéliales en réponse à l'infection par les spiroquettes.

Ainsi, un cinquième objet de l'invention concerne l'utilisation d'un produit dermatologique topique composé de naphtoquinones conjuguées visant le traitement de dermatoses bactériennes caractérisés par la formation des filaments. Ces produits sont obtenus à partir de pigments naphtoquinones d'origine naturelle ou de synthèse.

Dans un aspect particulier, l'invention concerne un procédé de préparation d'un produit dermatologique topique (sous forme de crème, gel, poudre ou spray) présentant des propriétés bactéricide, fongicide ou répulsive d'ectoparasites.

Ce produit peut être intégré dans une formulation colorante, ou dans un kit de coloration afin d'associer la propriété colorante à l'action répulsive d'insecte, à l'action antifongique ou encore à l'action bactéricide.

Le procédé consiste à faire réagir au moins un composé I en présence d'un agent oxydant, et un composé III. Lesdits composés I et III étant chimiocompatibles entre eux, dans un milieu neutre. Le pH devenant acide suite à l'addition de sels d'aminoacides, à une température comprise entre 30°C et 55°C (exceptionnellement jusqu'à 70°C), pendant une durée comprise entre 10 et 45 minutes, dans lequel :

- le composé I est un précurseur de colorant, ledit précurseur étant une naphtoquinone, par exemple une naphtoquinone naturelle telle que lawsone, juglone, lapachone, plumbagine, menadione, methoxy-naphthoquinones...
- le composé III est un catalyseur naturel polyfonctionnel, ledit catalyseur étant un thioaminoacide.

De manière optionnelle, le procédé peut aussi mettre en jeu un composé II choisi parmi les naphtoquinones, les quinones, les antraquinones, les indoles, les bis-indoles, les xanthones, les flavonoïdes, les pyranoanthocyanes, les crommènes, les iridoïdes, les préridines, les isoquinolines, les phanoxaline, les phenazines, les polyènes, les caroténoïdes et les chalcones.

L'oxydant peut par exemple être le peroxyde d'hydrogène.

De préférence, le procédé de préparation d'un colorant instantané et hydrosoluble selon l'invention consiste à faire réagir les parties hydrosolubles des plantes riches en précurseurs de colorants chromophores et/ou groupements auxochromes en tant que sources des composés I, et/ou II et/ou III.

Dans un mode de réalisation particulier du procédé, la température de la réaction est comprise entre 30°C et 55°C, de préférence entre 40°C et 55°C, de manière encore plus préférée entre 45°C et 50°C, pouvant varier en fonction de la nature et stabilité des composés mélangés.

Dans un mode de réalisation préféré, le pH de la réaction est compris entre 8 et 9.

Les colorants de copigmentation ainsi obtenus sont des naphtoquinones conjuguées à une thio-aminoacide et ont des propriétés bactéricides qui s'exercent par interaction avec les kératines, que ce soit les kératines de la peau, les cheveux ou des poils. Ils trouvent donc leur application en santé humaine ou animale.

Les colorants de copigmentation obtenus selon ce procédé peuvent être utilisés en tant qu'agent bactéricide, fongicide et répulsif par application directe sur la peau, les cheveux ou les poils.

Ils sont alors imprégnés directement sur les poils (kératine) des animaux atteints, notamment les bovins, les canins, ou les chats. L'utilisation chez l'homme est facilitée par l'imprégnation directe en tant que colorants capillaires. Ces produits d'utilisation millénaire en médecine ayurvédique ou phytothérapie sont sans risque pour la santé humaine ou des animaux.

Les colorants de copigmentation peuvent également être utilisés pour colorer des fibres naturelles ou des tissus naturels.

Cette coloration consiste en une imprégnation des fibres par les molécules naturelles actives présentes dans les colorants de copigmentation. Le tissu doit être choisit en fonction de la fonctions souhaité et climat local (ex pyjama). La fibre en question doit être vierge (non colorée) et d'origine naturelle, végétale (coton) ou animale (laine, soie).

Ces copigments sont composés en fonction de l'affinité augmenté par certains types de fibre utilisées (ex. coton, laine, soie, alparga etc).

Pour colorer des fibres naturelles délicates, des températures douces (30°C pour la soie ; 40°C pour le coton) doivent être utilisées pendant la coloration afin de ne pas dégrader ou modifier la fonction des pigments utilisés ou l'intégrité des fibres.La présente invention sera mieux comprise à la lecture des exemples qui suivent, fournis à titre d'illustration et ne devant en aucun cas être considérés comme limitant la portée de la présente invention.

## EXEMPLES

### Exemple 1 : Extraction d'un composé A - colorant obtenu à partir des feuilles broyées de Henné (*Lawsonia inermis*)

Les feuilles broyées de henné (10% du poids des fibres à colorer) sont réduites en une fine poudre par broyage ou macération (v/v). L'eau distillée (100 mL) est chauffée (65 à 75°C) ou portée à ébullition. L'eau est additionnée à la poudre broyée et le tout est agité pendant 15 à 30 min, jusqu'à homogénéisation. Une solution hydroalcoolique (5 à 30%) peut remplacer l'eau.

Afin d'extraire un maximum des composés, la pâte obtenue peut-être macérée jusqu'à homogénéisation. La pâte épaisse obtenue est laissée au repos entre 3 à 24 heures à température ambiante. Après 24 heures, la solution est filtrée afin d'éliminer le résidu insoluble. Cette étape, dite « catalyse enzymatique » permet la libération des composés actifs (colorants /copigments) de ses glycosides naturels. L'addition d'une solution alcaline ($NaHCO_3$ ou $NA_2CO_3$) peut inhiber l'action enzymatique et empêcher ou retarder la libération des ingrédients actifs (colorants/copigments). Ceci est donc déconseillée en fonction du type de matière première utilisé.

En général, les molécules ayant un pouvoir colorant sont solubilisées dans une phase aqueuse ou hydroalcoolique. Néanmoins, certains composés sont peu solubles ou insolubles dans l'eau. Dans ce cas, il est nécessaire de transformer les ingrédients.

### Exemple 2 : Extraction d'un composé II - extrait aqueux des graines broyées de café torréfié

Plusieurs matières d'origine naturelle ayant les caractéristiques d'un pigment ou copigment (colorants alimentaires, pigments naturels en cosmétique etc) peuvent être utilisés afin d'obtenir le composé I ou II

L'extraction des copigments auxiliaires (Composé III) peut être réalisée par infusion-filtration directe (exemple café) sans besoin d'un temps de pause. Il est aussi possible d'utiliser des colorants alimentaires disponibles dans le commerce.

Si nécessaire, la solution extractive hydrosolubles est séparée des parties insolubles par filtration et stockée. La solution hydrosoluble obtenue est maintenue à température entre 6 à 10 °C, ou 4 à 8°C afin d'éviter la contamination par des microorganismes.

### Exemple 3 : Préparation d'un colorant naturels

a. Réaction mise en œuvre

Le ou les composés de type I sont solubilisés dans un volume d'eau distillée. La solution est ensuite filtrée en papier ou coton afin d'éliminer les résidus insolubles.

Le ou les copigments (composés de type II) ainsi que l'agent catalyseur (composé de type III) sont introduits à température ambiante et sous agitation, puis la température est augmentée rapidement jusqu'à atteindre 45 - 50°C (maximum 70°C) et maintenue sous agitation pendant une courte période de temps (10 à 15 min). Le mélange est ensuite refroidi à température ambiante et sous agitation pendant 30 à 60 min.

Le colorant peut être utilisé immédiatement après refroidissement, conservé au réfrigérateur pour une utilisation à court terme, ou encore congelé pour lyophilisation postérieure.

La solution colorante peut ensuite être lyophilisée.

b. congélation des produits de réaction

Après refroidissement, la solution obtenue est soit utilisée immédiatement, soit congelée afin de maintenir toutes les capacités colorantes et odorantes des extraits de plantes.

c. Lyophilisation des colorants

Préalablement à la lyophilisation, les compositions colorantes congelés sont broyées en petits morceaux à l'aide d'un broyeur électrique et de façon rapide afin d'éviter leur décongélation. L'objectif est d'obtenir des morceaux plus ou moins homogènes. Ces glaçons brisés sont ensuite posés dans la chambre (ou ballon) du lyophilisateur.

Ces glaçons brisés ont été lyophilisés avec un lyophilisateur Labconco Freezone 4.5L. L'eau a été sublimée à une basse température (-50°C) et à un vide puissant (2.10-2 mbar) grâce à une pompe Alcatel. Les composés ont été laissés dans le lyophilisateur jusqu'à obtention d'un lyophilisat complètement sec.

Alternativement, les extraits hydrosolubles filtrés peuvent être directement utilisés pour la lyophilisation, sans l'étape de congélation préalable.

Le rendement du lyophilisats préparés et analysés varient entre 25 à 60% (pour certaines matières premières pouvant atteindre 80%) du poids de l'extrait brut, résultat dépendant du produit brut de départ et de la technique d'extraction utilisée.

$$\text{Rendement} = (\text{lyophilisat / extrait brut sec}) *100$$

Les lyophilisats/extraits secs sont conservés hermétiquement fermés jusqu'à leur utilisation, de préférence sous atmosphère inerte (argon) ou sous vide.

d. Séchage par atomisation

Une façon alternative moins coûteuse que la lyophilisation est le séchage par pulvérisation, le séchage d'un liquide pour obtenir une poudre. Cette technique est couramment utilisée pour l'obtention de lait, soupe, jus, ou café en poudre.

La solution hydrosoluble (SSR) est introduite par le haut sous pression, avec un jet d'air chaud. Les gouttelettes qui retombent sèchent pour donner une poudre fine qui refroidit en descendant. Les granules humides sont ensuite séchés à mesure qu'ils descendent par une deuxième tour et sont tamises pour obtenir des granules finaux de taille uniforme. Cette technique impose des conditions moins douces que la lyophilisation, pouvant conduire à une conservation moins efficace des composés actifs (colorants/copigments).

**Exemple 4 : Préparation d'un colorant pour application capillaire**

a. Colorants et copigments utilisés

Composés I et II : feuilles broyées de henné (*Lawsonia inermis*) et grains de café torréfié (Csp)
Composé III: Aminoacide (qualité alimentaire)

b. Réaction mise en œuvre

Les feuilles broyées de henné (25 g) sont mélangées avec l'eau distillée bouillante (100 mL à 150 mL, puis mélangées vigoureusement pendant 15 min et ensuite laissées au repos pendant 24 heures. La solution est ensuite filtrée dans un papier filtre ou tige à coton. L'extrait de café torréfié (5 g/30 mL d'eau) est rajouté à l'extrait de henné. Enfin, le composé B, solution aqueuse de thio-aminoacide (0,1 g) est rajouté au mélange henné-café.

Le mélange est chauffé jusqu'à 50°C (maximum 70°C), sous agitation, pendant 15 minutes et refroidi à température ambiante.

Le colorant instantané (lyophilisat henné-café-aminoacide) présente un aspect étoilé uniforme, couleur brune chocolaté (très différente du henné seul) et maintien les parfum de café de l'extrait original.

Pour des cheveux longs il faut prévoir au moins 50 g de poudre de henné et 10g de poudre de café.

## Exemple 5 : Application d'un coloration capillaire instantané

L'utilisation des lyophilisats au moment de l'application est très simple. Les étapes suivantes sont proposées :

i. Dissolution du lyophilisat dans un petit volume d'eau pour application directe ou préparation d'un masque.
ii. Préparation *in situ* d'un masque colorant :

◦ Solubilisation des lyophilisats des matières colorantes avec un petit volume d'eau (5 ml) et addition avec une spatule d'un beurre (ex : karité). Homogénéisation.
◦ Alternativement, le lyophilisat peut être dissous directement dans un gel hydrosoluble (ex : gel carraghénane 2%) ou beurre (ex : beurre de karité)
Le masque obtenu présente une consistance appropriée et les caractéristiques organoleptiques de départ sont maintenues, notamment la couleur et l'odeur.

iii. Application de la solution ou masque directement sur les cheveux secs

o Disposer le masque colorant en mèches sur le cuir chevelu à l'aide d'une brosse et distribution avec un peigne fin afin de couvrir l'ensemble de la chevelure.
o Le séchage (au sèche-cheveu) accélère la coloration et permet l'obtention de couleurs plus intenses.

iv. Repos de 30 min (minimum, idéalement 45 min)
v. Rinçage à l'eau tiède pour enlever excédent et sécher les cheveux normalement

Alternativement, les cheveux peuvent être lavés avec shampoing classique et rincés à l'eau tiède.

Le tableau 3 ci-dessous résume les étapes nécessaires pour l'application des colorants.

### TABLEAU 3 : Mode d'emploi des produits colorants

| Etape | Produits | Méthode | Résultat |
|---|---|---|---|
| i | Lyophilisats : Henné + Café + mordant (~ 7 g) Eau : 15 à 20 mL | Addition de l'eau dans le lyophilisat (température ambiante ou faiblement tiède) | Solution homogène (I). |
| ii | Gel carraghénane 0,5 à 2% | Addition du gel dans la solution I. Mélanger de façon manuelle jusqu'à obtention d'une consistance épaisse et homogène des couleurs | Masque (II) homogène |
| iii | Solution (I) OU Masque (II) | - Application sur les cheveux de I ou II<br>- Distribuer le produit à l'aide d'un peigne fin<br>-Séchage avec sèche-cheveux[a]<br>- Repos pendant 30 à 45 min<br>- Rincer à l'eau tiède ou shampoing | Cheveux brun chocolaté avec belles lumières, Nuance de couleurs originales, Parfum café suave |
| (a) L'objectif ici n'est pas de sécher les cheveux, mais d'accélérer l'imprégnation des colorants sur les fibres | | | |

## Exemple 6 : Test comparatif entre colorant naturel instantané selon l'invention *versus* colorant permanent

Un test comparatif de coloration a été réalisé avec les produits suivants :

• Colorant (X) acquis sur le commerce, ayant l'appellation *'coloration aux extraits végétaux'* composés des molécules de synthèse avec hydrolysats des protéines naturelles

• *Solution* colorante (Y) objet de l'invention, composé uniquement des colorants et pigments naturels et catalyseur (aminoacide ou son sel).

• Solution colorante (Z) recomposée à partir du lyophilisat obtenu avec la composition (Y)

**EP 3 740 187 B1**

Une étoffe de laine mouton (lavée et vierge), lavée au shampoing neutre et vierge (sans coloration préalable) a été utilisé comme modèle des cheveux humains.

| | Composé A | Support galénique | Kératine naturelle (TEST) | Couleur |
|---|---|---|---|---|
| **Colorant** (disponible sur le marché) * (nommé 'X') | Mélange des molécules synthétiques (dérivés du p- aminophénol, chlororesorcinol, amino-toluène, éthanolamine etc | Gel (15 ml) préparé in situ avec peroxyde d'oxygène (H$_2$0$_2$) | - Application sur fibre<br>- Repos 30 min<br>- Chauffé à 50°C | Brun cho-colaté avec des notes dorées (nommé blond foncé doré) |
| **Invention** : Colorants naturels selon l'invention (nommés « **Y** ») | • Henné (25 mg/ml)<br><br>• Extrait de café (200 mg/mL)<br><br>• Aminoacide (7mg) (pH acide) | Eau distillé | - Application sur fibre<br><br>- Chauffé à 50°C<br>- Repos 30 min | Brun cho-colaté |
| **Invention** : Colorants instantané (nommés « **Z** » ) | Lyophilisat (100 mg) de B | lyophilisat dissous dans l'eau ( 4 mL) | - Application sur fibre<br>- Chauffé à 50°C<br>- Repos 30 min | Brun cho-colaté |
| * Colorants actifs : solution contenant mélange molécules synthétiques qui sont mélangé in situ avec le peroxyde d'oxygène lors de l'application | | | | |

Les compositions en ingrédients actifs et adjuvants des produits de l'état de la technique et de ceux de l'invention sont très différentes.

| CARACTERISTIQUE | L'INVENTION | PRODUIT TEST |
|---|---|---|
| Origine des matières | 100% d'origine naturelle | Synthétiques & actifs naturels |
| Oxydants | Aucun (copigments naturels) | Oxydant (révélateur des couleurs) |
| Chaleur | 50°C | 50°C |
| Temps de pause | 30 min | 30 min |

**Exemple 7** : **Préparation d'un colorant à usage dermo-phytopharmaceutique**

En présence d'un agent oxydant (ex. H$_2$O$_2$) la molécule lawsone présente dans l'extrait de henné peut générer un dérivé conjugué ayant également des propriétés bactéricides et une plus grande affinité par les fibres de kératine. I, III 30 -50°C/10 - 15 min / pH 7-9 [H$_2$O$_2$] => Conjugué naphtoquinone

- Noyer/juglone [50 à100 g dissous dans d'eau distillé)
- Henné /lawsone [50 à100 g dissous dans d'eau distillé)
- Tannin : 2,5 à 10g
- Thio-aminoacide : 10 - 30 mg
- Eau distillé : qsp 30 ml

La solution réactionnelle obtenue est traitée par lyophilisation / séchage par atomisation est ensuite appliquée avec différents supports de formulation (beurre, gel etc) sur les cheveux ou sur la peau.

En présence d'un agent oxydant (ex. H$_2$O$_2$) la molécule lawsone présente dans l'extrait de henné peut générer un dérivé conjugué ayant également des propriétés bactéricides et une plus grande affinité par les fibres de kératine.

# EP 3 740 187 B1

## Revendications

1. Procédé de préparation d'un colorant instantané et hydrosoluble consistant à faire réagir au moins un composé I et un composé II avec au moins un composé III, ces trois composés étant chimio compatibles entre eux, dans un milieu faiblement acide dont le pH est compris entre 3,5 et 6,5 à une température comprise entre 30 ° C et 55 °C pendant une durée comprise entre 10 et 45 minutes, dans lequel :

   - le composé I est un précurseur de colorant,
   - le composé II est un copigment. Les composés I et II étant choisis parmi les naphtoquinones, les quinones, les anthraquinones, les indoles, les bis-indoles, les xanthones, les flavonoïdes, les pyranoanthocyanes, les chromènes, les iridoïdes, les ptéridines, les isoquinolines, les phénoxazines, les phénazines, les polyènes, les caroténoïdes et les chalcones ;
   - le composé III est un catalyseur naturel polyfonctionnel choisi parmi les tannins, les acides aliphatiques, les acides aromatiques et les acides phénoliques, en particulier un acide aminé ou son sel.

2. Procédé selon la revendication 1 dans lequel ledit composé III est un thioaminoacide.

3. Procédé selon l'une des revendications 1 ou 2 comprenant en outre une étape préalable, consistant à extraire ledit au moins un composé I ou II à partir d'extraits de plantes.

4. Procédé selon l'une des revendications 1 à 3 comprenant en outre une étape de lyophilisation ou de séchage par atomisation des produits colorants de sorte à obtenir une poudre instantanée.

5. Procédé selon l'une des revendications 1 à 4 dans lequel les composés I, II et III sont exclusivement d'origine naturelle.

6. Colorant de copigmentation obtenu par le procédé défini à l'une des revendications 1 à 5.

7. Colorant tel que défini à la revendication 6, **caractérisé en ce qu'**il se trouve sous forme liquide ou de poudre instantanée.

8. Colorant tel que défini à la revendication 6 **caractérisé en ce qu'**il est une poudre instantanée, hydrosoluble et qu'il ne comprend pas de fraction insoluble.

9. Procédé de préparation d'un colorant instantané et hydrosoluble consistant à faire réagir au moins un composé I et un composé III, lesdits composés I et III étant chimiocompatibles entre eux, dans un milieu faiblement basique dont le pH est compris entre 7,5 et 9, à une température comprise entre 30°C et 55°C, exceptionnellement jusqu'à 70°C, pendant une durée comprise entre 10 et 45 minutes, dans lequel :

   - le composé I est un précurseur de colorant, ledit précurseur étant une naphtoquinones, par exemple les naphtoquinones naturelles telle que lawsone, juglone, lapachone, plumbagine, menadione, methoxy-naphthoquinones.
   - le composé III est un catalyseur naturel polyfonctionnel, ledit catalyseur étant un thioaminoacide.

10. Colorant de copigmentation selon la revendication 9 **caractérisé en ce qu'**il s'agit d'une naphtoquinone conjuguée.

11. Kit de coloration capillaire comprenant :

    - des produits colorants tels que définis à l'une des revendications 6 à 8 et 10 sous forme de poudres instantanées (lyophilisats)
    - un gel ou un beurre à usage cosmétique
    - des ustensiles pour mélanger les composants et appliquer le masque obtenu.

12. Colorant selon revendication 6 à 8 et 10 pour son utilisation dans le domaine phytopharmaceutique, pharmaceutique et vétérinaire.

13. Colorant selon la revendication 10 pour son utilisation selon la revendication 12 par ses propriétés bactéricide, fongicide ou antiseptique pour application sur la peau, les cheveux, les poils ou les fibres naturelles de kératine ou

18

xénofibres.

14. Utilisation non-thérapeutique d'un colorant selon l'une des revendications 6 à 8 et 10 dans les domaines cosmétique, tatouage de la peau, agroalimentaire, phytopharmaceutique et dans le domaine de la teinture textile.

15. Utilisation d'un colorant selon la revendication 14 pour colorer les fibres ou tissus naturels.


**Patentansprüche**

1. Ein Verfahren zur Herstellung eines wasserlöslichen Sofortfarbstoffs umfasst die Umsetzung mindestens einer Verbindung I und einer Verbindung II mit mindestens einer Verbindung III, wobei diese drei Verbindungen chemokompatibel miteinander sind, in einem schwach sauren Medium mit einem pH-Wert zwischen 3,5 und 6,5 bei einer Temperatur zwischen 30 °C und 55 °C für einen Zeitraum zwischen 10 und 45 Minuten, wobei:

   - Verbindung I ein Farbstoffvorläufer ist,
   - Verbindung II ein Copigment ist. Die Verbindungen I und II werden ausgewählt aus Naphthochinonen, Chinonen, Anthrachinonen, Indolen, Bisindolen, Xanthonen, Flavonoiden, Pyranoanthocyanen, Chromenen, Iridoiden, Pteridinen, Isochinolinen, Phenoxazinen, Phenazinen, Polyenen, Carotinoiden und Chalkonen;

   - Verbindung III ist ein natürlicher polyfunktioneller Katalysator, ausgewählt aus Tanninen, aliphatischen Säuren, aromatischen Säuren und Phenolsäuren, insbesondere eine Aminosäure oder ihr Salz.

2. Verfahren nach Anspruch 1, wobei die Verbindung III eine Thioaminosäure ist.

3. Verfahren nach einem der Ansprüche 1 oder 2, das außerdem einen vorherigen Schritt umfasst, der aus der Extraktion mindestens einer Verbindung I oder II aus Pflanzen besteht.

4. Verfahren nach einem der Ansprüche 1 bis 3, das außerdem einen Schritt des Gefriertrocknens oder Sprühtrocknens von Farbstoffen umfasst, um ein Instantpulver zu erhalten.

5. Verfahren nach einem der Ansprüche 1 bis 4, bei dem die Verbindungen I, II und III ausschließlich natürlichen Ursprungs sind.

6. Co-Pigmentierungsfarbstoff, erhältlich nach dem in einem der Ansprüche 1 bis 5 definierten Verfahren.

7. Farbstoff nach Anspruch 6, **dadurch gekennzeichnet, dass** er in flüssiger Form oder als Instantpulver vorliegt.

8. Farbstoff nach Anspruch 6, **dadurch gekennzeichnet, dass** es sich um ein wasserlösliches Instantpulver handelt und dass es keinen unlöslichen Anteil enthält.

9. Ein Verfahren zur Herstellung eines sofort einsatzbereiten, wasserlöslichen Farbstoffs umfasst die Umsetzung mindestens einer Verbindung I und einer Verbindung III, wobei die Verbindungen I und III chemisch miteinander kompatibel sind, in einem schwach basischen Medium mit einem pH-Wert zwischen 7,5 und 9 bei einer Temperatur zwischen 30 °C und 55 °C, in Ausnahmefällen bis zu 70 °C, für einen Zeitraum zwischen 10 und 45 Minuten, wobei:

   - Verbindung I ein Farbstoffvorläufer ist, wobei der Vorläufer ein Naphthochinon ist, beispielsweise natürliche Naphthochinone wie Lawson, Juglon, Lapachon, Plumbagin, Menadion oder Methoxynaphthochinone.
   - Verbindung III ein natürlicher polyfunktioneller Katalysator ist, wobei der Katalysator eine Thioaminosäure ist.

10. Copigmentierungsfarbstoff nach Anspruch 9, **dadurch gekennzeichnet, dass** es sich um ein konjugiertes Naphthochinon handelt.

11. Haarfärbeset, bestehend aus:

   - Farbprodukten gemäß einem der Ansprüche 6 bis 8 und 10 in Form von Instantpulvern (Lyophilisaten)
   - einem Gel oder einer Butter für kosmetische Zwecke
   - Utensilien zum Mischen der Komponenten und Auftragen der resultierenden Maske.

**12.** Farbstoff nach Anspruch 6 bis 8 und 10 für seine Verwendung im phytopharmazeutischen, pharmazeutischen und veterinärmedizinischen Bereich.

**13.** Farbstoff nach Anspruch 10 für seine Verwendung gemäß Anspruch 12 aufgrund seiner bakteriziden, fungiziden oder antiseptischen Eigenschaften zur Anwendung auf Haut, Haaren, Fell oder natürlichen Keratinfasern oder Xenofasern.

**14.** Nicht-therapeutische Verwendung eines Farbstoffes nach einem der Ansprüche 6 bis 8 und 10 in den Bereichen Kosmetik, Hauttätowierung, Agrar- und Ernährungswirtschaft, Phytopharmaka und im Bereich der Textilfärbung.

**15.** Verwendung eines Farbstoffs gemäß Anspruch 14 zum Färben von Naturfasern oder Textilien.

**Claims**

**1.** Process for the preparation of a water-soluble instant dye consisting in reacting at least one compound I and one compound II with at least one compound III, these three compounds being chemocompatible with each other, in a weakly acidic medium whose pH is between 3,5 and 6.5 at a temperature between 30 ° C and 55 °C, for a period of time between 10 and 45 minutes, wherein:

- compound I is a dye precursor,
- compound II is a copigment. The compounds I and II being chosen from naphtoquinones, quinones, anthraquinones, indoles, bis-indoles, xanthones, flavonoids, pyranoanthocyanins, chromens, iridoids, pteridines, isoquinolines, phenoxazine, phenazines, polyenes, carotenoids and chalcones;
- compound III is a polyfunctional natural catalyst selected from tannins, aliphatic acids, aromatic acids and phenolic acids, in particular an amino acid or its salt.

**2.** The process of claim 1 in which said compound III is a thioamino acid.

**3.** Process according to one of claims 1 or 2 further comprising a prior step of extracting at least one compound I or II from plants extracts.

**4.** Process according to one of claims 1 to 3 further comprising a step of lyophilization or spray drying of the dyestuffs so as to obtain an instant powder.

**5.** Process according to one of claims 1 to 4 in which the compounds I, II and III are exclusively of natural origin.

**6.** Copigmentation dye obtained by the process defined at one of claims 1 to 5.

**7.** Dyestuff as defined in the claim 6, **characterized by** its form liquid or instant powder.

**8.** Dyestuff as defined in claim 6, **characterized in that** it is instant powder, water-soluble and that it does not include an insoluble fraction.

**9.** Process for preparing a water-soluble instantaneous dye, the process consisting in reacting at least one compound I and one compound III, said compounds I and III being chemocompatible with each other in a weakly basic medium whose pH is between 7.5 and 9, at a temperature between 30 ° C and 55 ° C, exceptionally up to 70° C, for a period between 10 and 45 minutes, wherein:

- the compound I is a dye precursor, said precursor being a naphthoquinone, for example a natural naphthoquinone such as lawsone, juglone, lapachone, plumbagine, menadione, methoxy-naphthoquinones.
- compound III is a polyfunctional natural catalyst, said catalyst being a thio-amino acid.

**10.** Copigmentation dye according to claim 9 **characterized in that** it is a conjugated naphthoquinone.

**11.** Hair coloring kit comprising:

- coloring products as defined in one of claims 7 to 8 and 10 under instantaneous powder (lyophilisates)

- a gel or a butter for cosmetic use
- utensils to mix the components and apply the mask obtained.

12. Dye according to one of claims 6 to 8 and 10 for use in the field of phytopharmaceutical, pharmaceutical and veterinary.

13. Dyestuff according to claim 10 for its use according to claim 12 for its bactericidal, fungicide or antiseptic properties for application to the skin, hair, velus hair, natural keratin fiber or xenofibers.

14. Non-therapeutic use of a dye according to one of claims 6 to 8 and 10 in the cosmetic field, for skin tattooing, in the agri-food, phytopharmaceuticals industry and in the field of textile dyeing.

15. Use of a dye according to claim 14 for coloring natural tissues or fibers.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- *Food and Chemical Toxicology*, 2004, vol. 42, 517-543
- *CHEMICAL ABSTRACTS*, 84.988-66-9
- *Contact Dermatitis*, 2006, vol. 55, 26-29
- *Nat Prod Res.*, 2014, vol. 28 (11), 835-7
- *J Agric Food Chem.*, 12 December 2012
- *Nat Prod Res.*, 2012, vol. 26 (12), 1119-24
- *Phytother Res.*, December 2001, vol. 15 (8), 676-80
- **CHELOSSI, E. ; FAIMALI, M.** *Science of The Total Environment*, 2006, vol. 356, 1-10
- **KAPADIA, J. G et al.** *Anti-Cancer Agents in Medicinal Chemistry- Anti-Cancer Agents*, 2013, vol. 13, 1500-1507
- **YUSUF, M. et al.** *Journal of Cleaner Production*, 2012, vol. 27, 42-50
- Henne. Absorption de la lawsone par le cheveau. **FORESTIERT et al.** Int. J. of Cosmetic Sci.. Kluver Academic Publishers, 01 January 1982, vol. 4, 153-174
- **M. J MIDDELVEEN ; P. J MAYNE ; D. G KAHN ; R. B STRICKER.** Characterization and evolution of dermal filaments. *Clin Cosmet Investig Dermatol*, 2013, vol. 6, 1-21
- **MIDDELVEEN MJ ; ROTARU GM ; MCMURRAY JL ; FILUSH KR ; SAPI E et al.** Canine Filamentous Dermatitis Associated with Borrelia Infection. *J Vet Sci Med Diagn*, 2016, vol. 5, 6
- **PRADEEPA V ; SENTHIL-NATHAN S ; SATHISH-NARAYANAN S3 et al.** Potential mode of action of a novel plumbagin as a mosquito repellent against the malarial vector Anopheles stephensi. *; Pestic Biochem Physiol.*, November 2016, vol. 134, 84-93
- **MOHD et al.** Assessment of colorimetric, antibacterial and antifungal properties of woollen yarn dyed with the extract of the leaves of henna (Lawsonia inermis). *Journal of Cleaner Production*, 2012, vol. 27 (42), 50
- *J Dtsch Dermatol Ges*, April 2010, vol. 8 (4), 234-42
- *Clin Cosmet Investig Dermatol.*, 2013, vol. 6, 1-21
- **MIDDELVEEN MJ.** *Stricker RB*, 2011